# EUROPÄISCHE PATENTSCHRIFT

(11) **EP 0 709 359 B1**
(45) Veröffentlichungstag und Bekanntmachung des Hinweises auf die Patenterteilung: **17.06.1998**
(21) Anmeldenummer: 95116724.6
(22) Anmeldetag: 24.10.1995
(51) Int. Cl.: C07C 43/178, C07C 41/08

(54) **Verfahren zur Herstellung von Monovinylethern**
Process for the preparation of monovinyl ethers
Procédé pour la préparation d'éthers monovinyliques

(30) Priorität: 29.10.1994 DE 4438707
(43) Veröffentlichungstag der Anmeldung: 01.05.1996
(73) Patentinhaber: BASF AKTIENGESELLSCHAFT, 67056 Ludwigshafen (DE)
(72) Erfinder: Heider, Marc, Dr., D-67433 Neustadt (DE); Rühl, Thomas, Dr., D-67227 Frankenthal (DE); Helfert, Herbert, Dr., D-67227 Frankenthal (DE); Schmidt-Radde, Martin, Dr., D-67259 Beindersheim (DE); Henkelmann, Jochem, Dr., D-68165 Mannheim (DE)

(56) Entgegenhaltungen:
- DE-A- 2 628 408
- DE-C- 958 383
- FR-A- 1 159 861
- US-A- 1 959 927
- US-A- 3 657 360
- JUSTUS LIEBIGS ANNALEN DER CHEMIE, Bd. 601, 1956 WEINHEIM, Seiten 81-111, W. REPPE 'Vinylierung'
- CHEMICAL ABSTRACTS, vol. 48, no. 2, 25.Januar 1954 Columbus, Ohio, US; M. F. SHOSTAKOVSKII ET AL 'Vinylation of ethylene glycol. Ethylene glycol mono-, divinyl ethers. Ethylene glycol cyclic acetal' Spalte 586g;

## Beschreibung

Die vorliegende Erfindung betrifft ein verbessertes Verfahren zur Herstellung von Monovinylethern der allgemeinen Formel I 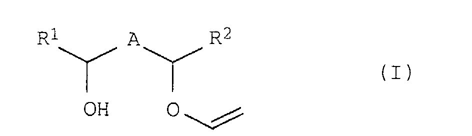 in der A für eine chemische Bindung, eine Methylen- oder Ethylengruppe steht, die Reste R¹ und R² unabhängig voneinander für Wasserstoff, Alkyl-, Cycloalkyl- oder Arylgruppen stehen oder R¹ und R² gemeinsam eine C₃-C₅-Alkylenbrücke bilden, durch Umsetzung eines Diols der allgemeinen Formel II 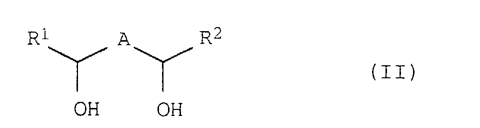 in der die Variablen die oben angegebene Bedeutung haben, mit Acetylen in Gegenwart einer Base bei einem Acetylenpartialdruck von 5 bis 25 bar bis zu einem Umsatz des Diols von 40 bis 80 %.

Es ist durch Reppe et al. (Liebigs Ann. Chem. 601 (1956) 81) bekannt, daß die Umsetzung von Diolen der Formel II mit Acetylen zu Gemischen der entsprechenden Monovinylether, der Divinylether (durch zweifache Reaktion der Hydroxylgruppen mit Acetylen) und cyclischer Acetale führt. Die Acetale entstehen insbesondere aus den Monovinylethern von 1,2-Diolen unter Ausbildung fünfgliedriger Cyclen.

Die Druckschrift lehrt die Vinylierung von Ethylenglykol bei 120°C und 20 bar Acetylendruck. Die Raum-Zeit-Ausbeute ist mit 15,8 g Monovinylether/l·h für eine großtechnische Anwendung unbefriedigend. Bei einer Reaktionstemperatur von 180°C wurde das unerwünschte cyclische Glykolacetal zum Hauptprodukt der Umsetzung.

Die DE-A 958 383 betrifft die Herstellung von Monovinylethern von Diolen bei Reaktionstemperaturen über 180°C und Normaldruck. Dabei wird der gebildete Monovinylether durch Einleiten überschüssigen Acetylens aus der Reaktionsmischung ausgetrieben und anschließend isoliert. Diese Verfahrensweise erfordert den Einsatz eines beträchtlichen Acetylenüberschusses. Das Acetylen muß zum wirtschaftlichen Betreiben einer entsprechenden Anlage nach der Isolierung des Monovinylethers gereinigt und in die Reaktion rückgeführt werden. Eine solche Verfahrensweise ist technisch aufwendig und daher unerwünscht.

Mikhanteva et al. (Zh. Prikl. Khim. 63 (1990) 172) beschreiben die Vinylierung von Diolen bei 145°C und 1 bar Druck. Die Monovinylether werden als unter den Reaktionsbedingungen instabil bezeichnet; sie reagieren weiter zu den Divinylethern oder zu den entsprechenden cyclischen Acetalen.

Es bestand die Aufgabe, ein Verfahren zur Verfügung zu stellen, das die Herstellung von Monovinylethern in hoher Selektivität erlaubt. Der Anteil an cyclischen Nebenprodukten soll dabei gering sein. Insbesondere war es Aufgabe der vorliegenden Erfindung, ein Verfahren mit einer hohen Raum-Zeit-Ausbeute zu finden.

Demgemäß wurde das oben definierte Verfahren zur Herstellung von Monovinylethern der Formel I gefunden, das dadurch gekennzeichnet ist, daß man die Umsetzung bis zu einem Umsatz des Diols von 40 bis 80 % bei 150 bis 250°C vornimmt, die Reaktion abbricht und den Monovinylether der Formel I isoliert.

Als Ausgangsverbindungen für das Verfahren kommen Diole der Formel II in Betracht. Die Variable A kann darin für eine chemische Bindung, eine Methylen- oder eine Ethylengruppe stehen.

Die Reste R¹ und R² stehen unabhängig voneinander für Wasserstoff, Alkylgruppen, vorzugsweise C₁-C₆-Alkyl wie Methyl, Ethyl und n-Propyl, weiterhin Cycloalkylgruppen, vorzugsweise C₄-C₇-Cycloalkyl wie Cyclopentyl und Cyclohexyl oder eine Arylgruppe, wobei C₆-C₁₀-Aryl wie Phenyl bevorzugt ist. Die Arylgruppen können einen bis drei inerte Substituenten wie Alkyl-, Alkoxy- oder Halogenreste tragen. Außerdem können die Reste R¹ und R² gemeinsam eine C₃-C₅-Alkylengruppe bilden.

Als Diole der Formel II seien genannt: Ethylenglykol, 1,2-Propylenglykol, 1,3-Propylenglykol, 1,2-Butandiol, 1,3-Butandiol, 1,4-Butandiol, 2,5-Hexandiol, 2,3-Hexandiol und 1,2-Cyclohexandiol. Bevorzugt werden Ethylenglykol, 1,2-Propylenglykol und 1,4-Butandiol.

Diese Diole sind im Handel erhältlich oder in an sich bekannter Weise aus den entsprechenden Epoxiden durch Ringöffnung mit Alkalimetallhydroxiden erhältlich.

Acetylen kann unverdünnt oder mit Inertgasen wie Stickstoff, Methan, Propan oder Argon verdünnt eingesetzt werden. Acetylen wird im allgemeinen in Mengen von 0,4 bis 5 mol pro Mol Diol eingesetzt.

Als Basen kommen im Prinzip alle Verbindungen in Betracht, die in der Lage sind, aus den Diolen der Formel II das entsprechenden Monoalkoholat zu bilden.

Insbesondere sind folgende Verbindungsklassen zu nennen:
- Alkalimetallhydride wie NaH und KH;
- Alkalimetallamide wie Natriumamid und Kaliumamid;
- Alkalimetall- und Erdalkalimetallhydroxide wie NaOH, KOH, CsOH und Ca(OH)₂;
- Alkalimetallalkoholate wie Natriummethylat und Kaliummethylat.
Bevorzugt wird Kaliumhydroxid.

Die Basen können in katalytischen Mengen eingesetzt werden, in der Regel genügen 0,005 bis 0,05 mol pro Mol Diol.

In einer bevorzugten Ausführungsform werden Diol und Kaliumhydroxid unter Abdestillieren von Wasser erhitzt, wobei sich das Monokaliumsalz des Diols bildet. Dann wird zu dieser Mischung Acetylen gegeben.

Die Reaktion kann in einem inerten Lösungsmittel wie THF oder Diethylenglykoldimethylether durchgeführt werden; bevorzugt wird aber kein Lösungsmittel zugesetzt.

Die Reaktionstemperatur beträgt 150 bis 250°C, bevorzugt 150 bis 180°C. Oberhalb dieser Temperaturen bildet sich vermehrt das unerwünschte Acetal, unterhalb kann keine befriedigende Raum-Zeit-Ausbeute erzielt werden.

Der Acetylenpartialdruck beträgt 5 bis 25 bar, bevorzugt 10 bis 20 bar. Unterhalb dieser Bereiche verläuft die Reaktion zu langsam, um akzeptable Reaktionszeiten zu erzielen. Höhere Drücke, die sich technisch nur aufwendig realisieren lassen, bringen keine erkennbaren Vorteile.

Die Umsetzung wird bei einem Umsatz von 40 bis 80 % des Diols abgebrochen. Der Umsatz kann leicht etwa durch Entnahme einer Probe und deren gaschromatographische Analyse bestimmt werden.

Je nach den gewählten Verfahrensbedingungen liegen die Reaktionszeiten im allgemeinen bei 1 bis 20 h.

Die Reaktion kann sowohl kontinuierlich wie auch diskontinuierlich durchgeführt werden. Dazu kommen Reaktoren wie Rührautoklaven oder Rohrreaktoren in Betracht.

Ist ein Umsatz von 40 bis 80 % des Diols erreicht worden, kann die Reaktion durch Entspannen des Reaktionsgemisches abgebrochen werden. Aus dem Reaktionsaustrag wird das Verfahrensprodukt I in der Regel destillativ isoliert. Nicht umgesetztes Diol der Formel II kann nach Isolierung in die Reaktion zurückgeführt werden.

Die vorliegende Erfindung erlaubt die Herstellung der Monovinylether I in hoher Selektivität mit guten Raum-Zeit-Ausbeuten.

Die Verfahrensprodukte I dienen als Zwischenprodukte für die Herstellung strahlungshärtender Lacke. Auch die als Nebenprodukt erhaltenen Divinylether können als vernetzbare Monomere für strahlungshärtbare Lacke verwendet werden.

### Beispiele

### Beispiel 1

In einen 20 l-Hochdruckautoklaven wurde eine Mischung aus 14 kg Ethylenglykol und 450 g KOH, die zuvor zur Entfernung von 150 g Wasser in 2 h im Vakuum destilliert war, eingefüllt. Mit Stickstoff wurde ein Druck von 2 bar angelegt. Der Autoklav wurde auf die in Tabelle 1 angegebene Temperatur aufgeheizt. Anschließend wurde Acetylen bis zu einem Gesamtdruck von 20 bar nachgepreßt. Verbrauchtes Acetylen wurde durch Nachpressen ersetzt. Nach beendeter Reaktion wurde abgekühlt, entspannt und der Reaktionsaustrag destilliert. Tabelle 1 gibt die Ergebnisse für verschiedene Reaktionstemperaturen wieder (S steht für Selektivität bzgl. des genannten Reaktionsprodukts).

**Tabelle 1**

| Temperatur [°C] | Laufzeit [h] | Umsatz an Diol [%] | S (Monovinylether) [%] | S (Divinylether) [%] | S (Acetal) [%] | Raum-Zeit-Ausbeute [g/l/h] |
|---|---|---|---|---|---|---|
| 130 (Vergleich) | 58 | 57 | 76 | 19 | 5 | 7,42 |
| 150 | 20 | 71 | 65 | 26 | 9 | 22,93 |
| 160 | 12 | 68 | 67 | 23 | 10 | 37,72 |
| 170 | 10 | 51 | 68 | 15 | 17 | 34,46 |

Bei 130°C wurde nur eine unbefriedigende Raum-Zeit-Ausbeute erzielt.

### Beispiel 2

Analog zu Beispiel 1 wurden in einem 300 ml-Hochdruckautoklaven 100 g 1,2-Propandiol und 5 g KOH umgesetzt. Tabelle 2 gibt die Ergebnisse wieder.

**Tabelle 2**

| Temperatur [°C] | Laufzeit [h] | Umsatz an Diol [%] | S (Monovinylether) [%] | S (Divinylether) [%] | S (Acetal) [%] | Raum-Zeit-Ausbeute [g/l/h] |
|---|---|---|---|---|---|---|
| 130 (Vergleich) | 62 | 66,8 | 78,1 | 12,5 | 9,4 | 3,37 |
| 150 | 6 | 80,6 | 75,4 | 13,3 | 11,3 | 40,5 |
| 160 | 3,5 | 67,4 | 77,4 | 12,2 | 10,4 | 59,6 |
| 170 | 3,5 | 73,1 | 58,2 | 12,3 | 29,5 | 48,6 |

### Beispiel 3

Ein 6 l-Rohrreaktor wurde mit Ethylenglykol gefüllt, in dem 2,62 Gew.-% KOH mit nachfolgender Entwässerung gelöst waren. Bei 160°C und 20 bar Druck wurden stündlich 450 l Acetylen über eine Strahldüse und 1,2 kg Ethylenglykol/KOH mit obiger Zusammensetzung über eine Hochdruckpumpe in den Reaktor eingebracht. Der kontinuierliche Überlauf wurde entspannt und analysiert.

| | |
|---|---|
| Umsatz: | 60 % |
| Selektivität (Monovinylether): | 59 % |
| Selektivität (Divinylether): | 35 % |
| Selektivität (Acetal): | 6 % |
| Raum-Zeit-Ausbeute (Monovinylether): | 70,8 g/l/h |

## Patentansprüche

1. Verfahren zur Herstellung von Monovinylethern der allgemeinen Formel I 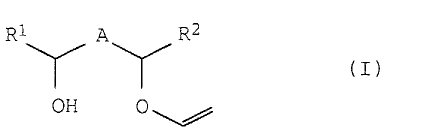 in der A für eine chemische Bindung, eine Methylen- oder Ethylengruppe steht, die Reste R¹ und R² unabhängig voneinander für Wasserstoff, Alkyl-, Cycloalkyl- oder Arylgruppen stehen oder R¹ und R² gemeinsam eine C₃-C₅-Alkylenbrücke bilden, durch Umsetzung eines Diols der allgemeinen Formel II 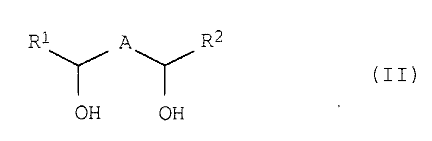 in der die Variablen die oben angegebene Bedeutung haben, mit Acetylen in Gegenwart einer Base bei einem Acetylenpartialdruck von 5 bis 25 bar bis zu einem Umsatz des Diols von 40 bis 80 %, dadurch gekennzeichnet, daß man die Umsetzung bis zu einem Umsatz des Diols von 40 bis 80 % bei 150 bis 250°C vornimmt, die Reaktion abbricht und den Monovinylether der Formel I isoliert.

2. Verfahren nach Anspruch 1, dadurch gekennzeichnet, daß man Ethylenglykol, 1,2-Propylenglykol oder 1,4-Butandiol umsetzt.

3. Verfahren nach Anspruch 1 oder 2, dadurch gekennzeichnet, daß die Reaktionstemperatur 150 bis 180°C beträgt.

4. Verfahren nach den Ansprüchen 1 bis 3, dadurch gekennzeichnet, daß man Kaliumhydroxid oder ein Kaliumalkoholat als Base verwendet.

## Claims

1. A process for preparing monovinyl ethers of the general formula I 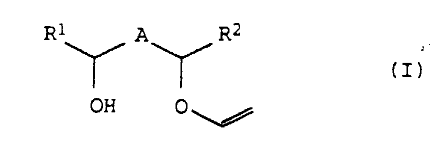 in which A is a chemical bond, a methylene group or ethylene group, the radicals R¹ and R² independently of one another are hydrogen, alkyl groups, cycloalkyl groups, aryl groups or R¹ and R² together form a C₃-C₅-alkylene bridge, by reacting a diol of the general formula II 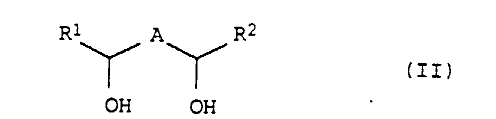 in which the variables have the meaning indicated above with acetylene in the presence of a base at an acetylene partial pressure of from 5 to 25 bar up to a conversion of the diol of from 40 to 80%, wherein the reaction is conducted up to a conversion of the diol of from 40 to 80% at from 150 to 250°C, the reaction is terminated and the monovinyl ether of the formula I is isolated.

2. A process as claimed in claim 1, wherein ethylene glycol, 1,2-propylene glycol or 1,4-butanediol is reacted.

3. A process as claimed in claim 1 or 2, wherein the reaction temperature is from 150 to 180°C.

4. A process as claimed in claims 1 to 3, wherein potassium hydroxide or a potassium alcoholate is used as base.

## Revendications

1. Procédé de préparation d'éthers monovinyliques de formule générale I 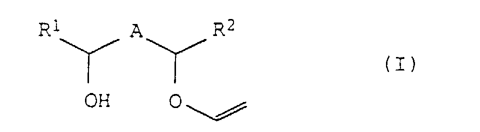 dans laquelle A est mis pour une liaison chimique, un groupement méthylène ou éthylène, les restes R¹ et R² représentent indépendamment l'un de l'autre des atomes d'hydrogène, des groupements alkyle, cycloalkyle ou aryle, ou bien R¹ et R² forment ensemble un pont alkylène en C₃-C_{5,} par réaction d'un diol de formule générale II 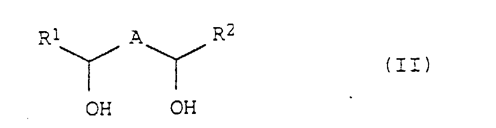 dans laquelle les variables prennent la signification susmentionnée, avec de l'acétylène en présence d'une base avec une pression partielle en acétylène de 5-25 bar, jusqu'à un taux de conversion du diol de 40-80%, caractérisé en ce que l'on mène la réaction jusqu'à un taux de conversion du diol de 40-80% à 150-250°C, on interrompt la réaction et on isole l'éther monovinylique de formule I.

2. Procédé selon la revendication 1, caractérisé en ce que l'on fait réagir de l'éthylèneglycol, du 1,2-propylèneglycol ou du 1,4-butanediol.

3. Procédé selon la revendication 1 ou 2, caractérisé en ce que la température réactionnelle vaut 150-180°C.

4. Procédé selon l'une quelconque des revendications 1 à 3, caractérisé en ce que l'on utilise de l'hydroxyde de potassium ou un alcoolate de potassium, en tant que base.
